# EUROPÄISCHE PATENTSCHRIFT

(11) **EP 1 409 474 B1**
(45) Veröffentlichungstag und Bekanntmachung des Hinweises auf die Patenterteilung: **05.03.2008**
(21) Anmeldenummer: 02719764.9
(22) Anmeldetag: 05.02.2002
(51) Int. Cl.: C07D 311/80

(54) **VERFAHREN ZUR HERSTELLUNG VON DRONABINOL**
METHOD FOR THE PRODUCTION OF DRONABINOL
PROCEDE DE PREPARATION DE DRONABINOL

(30) Priorität: 09.02.2001 DE 10106024
(43) Veröffentlichungstag der Anmeldung: 21.04.2004
(73) Patentinhaber: Steup, Christian, 65719 Hofheim (DE)
(72) Erfinder: Steup, Christian, 65719 Hofheim (DE)
(74) Vertreter: Pohlmann, Bernd Michael
(86) Internationale Anmeldenummer: PCT/EP2002/001172
(87) Internationale Veröffentlichungsnummer: WO 2002/062782

(56) Entgegenhaltungen:
- US-A- 5 227 537

## Beschreibung

Gegenstand der Erfindung ist ein Verfahren zur Herstellung von Dronabinol aus Faserhanf.

Dronabinol ist ein Cannabinoid mit der chemischen Bezeichnung (6aR-trans)-6a,7,8,10a-tetrahydro-6,6,9-trimethyl-3-pentyl-6H-dibenzo[b,d]pyran-1-ol. Dronabinol (= Δ9-Tetrahydrocannabinol) ist ein natürlicher Bestandteil verschiedener Cannabis-Pflanzen, aus denen er durch Extraktion gewonnen werden kann. Dronabinol kann auch chemisch synthetisiert werden und ist ein blass-gelbes, harziges Öl, das bei Raumtemperatur klebrig ist und bei kühler Lagerung hart wird. Es ist im Wasser unlöslich, kann aber in Ölen wie Sesamöl gelöst werden.

Aus dem US-Patent 4 025 516 ist bereits ein Verfahren zur Herstellung von Δ9-Tetrahydrocannabinol durch Kondensation des (+)-p-Mentha-2,8-dien-1-ol mit Olivetol in Gegenwart von BF3-Etherat bekannt. In der US-Patentschrift 5 342 971 ist außerdem die Synthese von Dronabinol aus Cannabidiolsäureestern in Gegenwart von Lewis-Säuren und anschließender Hydrolyse beschrieben. Darüber hinaus ist auch schon in der US-Patentschrift 5 227 537 die Synthese von Dronabinol aus Hydroxy-Cannabidiol mit Lewis-Säuren bekannt. Die Herstellung von Dronabinol aus Tetrahydrocarbinol-reichem Cannabis mit anschließender Destillation und/oder Chromatographie ist in der internationalen Patentanmeldung WO 00/25127 beschrieben.

Aufgabe der vorliegenden Erfindung ist, einen neuen und einfachen Zugang zu dem für medizinische Zwecke immer wichtiger werdenden Dronabinol zu finden.

Diese Aufgabe wird mit den Merkmalen der unabhängigen Patentansprüche gelöst.

Die vorstehend genannten synthetischen Verfahren sind entwickelt worden, weil eine Isolierung des Δ9-Tetrahydrocannabinols aus Cannabis indica gesetzlich verboten ist, z. B. durch die Bestimmungen des deutschen Betäubungsmittelgesetzes. Da die synthetischen Verfahren jedoch sehr aufwendig sind, andererseits Dronabinol wegen seiner immunsuppressiven und zytostatischen Eigenschaften, vor allem aber zur Linderung der bei der Krebstherapie auftretenden Nebenwirkungen zunehmende medizinische Bedeutung gewinnt, stellte sich die Aufgabe, ein einfaches Verfahren zur Herstellung von Dronabinol aus Pflanzenmaterialien zu gewinnen, das im Einklang mit den zu beachtenden gesetzlichen Bestimmungen steht. Als zulässig wird die Gewinnung des Δ9-Tetrahydrocannabinols aus dem Faserhanf (Cannabis sativa) angesehen.

Bei dem erfindungsgemäßen Verfahren zur Synthese von Dronabinol ist vorgesehen, dass man
- als Ausgangsstoffe für die Synthese von Dronabinol, Cannabidiolsäure und/oder Cannabidiol verwendet,
- das gegebenenfalls durch Decarboxylierung gewonnene Cannabidiol mit Hilfe von Lewis-Katalysatoren zu Dronabinol zyklisiert, um Rohdronabinol zu erhalten.

Gegenstand der Erfindung ist ferner ein Verfahren zur Herstellung von Dronabinol, bei dem man
a) Cannabidiol oder Cannabidiolsäure aus Faserhanf isoliert,
b) das ggf. durch Decarboxyierung gewonnene Cannabidiol in Gegenwart von Lewis-Säuren in einem nicht polaren Lösungsmittel zum Dronabinol zyklisiert,
c) dieses durch ein chromatographisches Verfahren isoliert und
d) den aus dem Eluat nach Abdestillation des Lösungsmittels gewonnenen Rückstand durch Vakuumdestillation reinigt.

Zur erfindungsgemäßen Herstellung von Dronabinol werden vorteilhafterweise zunächst die Blüten oder auch die Blätter des aus zertifiziertem Saatgut erzeugten Faserhanfs vom übrigen Pflanzenmaterial abgetrennt und nach dem Trocknen gesiebt. Es ist aber auch möglich, das von den Stengelteilen befreite, geschnittene Pflanzenmaterial ohne vorheriges Sieben für die Extraktion der Wirkstoffe einzusetzen. Da die Cannabidiolsäure und das Cannabidiol vor allem in den auf der Blatt- oder Blütenoberfläche angeordneten Harzdrüsen angereichert sind, und diese Drüsen durch das Sieben mechanisch vom restlichen Pflanzenmaterial abgetrennt werden können, gelingt es auf diesem Weg, ein Material zu gewinnen, dass die als Ausgangsstoffe für die Dronabinol-Synthese erforderliche Cannabidiolsäure und das Cannabidiol in recht hohen Konzentrationen enthält.

Aus den durch das Sieben gewonnenen Blütenbestandteilen oder dem geschnittenen Pflanzenmaterial werden erfindungsgemäß dann die Wirkstoffe durch Extraktion mit einem nicht-polaren Lösungsmittel, z. B. Benzin, isoliert. Zweckmäßigerweise erfolgt die Extraktion der Wirkstoffe in der Weise, dass man frische Blüten oder frisches Pflanzenmaterial mit einem Lösungsmittel behandelt, das bereits durch einen früheren Extraktionsvorgang mit Wirkstoffen angereichert ist. Auf diese Weise lässt sich die Wirkstoffkonzentration im Lösungsmittel kontinuierlich erhöhen, ohne dass eine Abdestillation des Lösungsmittels erforderlich ist. Da die Lösungsmittelbehandlung des Pflanzenmaterials bei Zimmertemperatur vorgenommen werden kann, wird damit eine thermische Decarboxylierung der aus den Blütenbestandteilen isolierten Cannabidiol-Säure weitgehend vermieden.

In dem so gewonnenen Lösungsmittel-Extrakt liegen je nach Alter und Vorbehandlung des Pflanzenmaterials Cannabidiol und Cannabidiol-Säure häufig nebeneinander vor. Während aus frischem Pflanzenmaterial ganz überwiegend die Cannabidiol-Säure gewonnen wird, überwiegt bei älterem Pflanzenmaterial das Cannabidiol.

Die weiteren Schritte zur Gewinnung des Cannabidiols hängen nun davon ab, ob in dem Blütenextrakt vorwiegend die Cannabidiol-Säure oder das Cannabidiol vorliegt.

Zur Gewinnung der Cannabidiol-Säure aus dem Lösungsmittelextrakt wird dieser mit einer wässrigen Lauge, vorzugsweise in einer Gegenstromextraktion in Gegenwart eines Reduktionsmittels, behandelt, wobei die Cannabidiol-Säure unter Salzbildung in der wässrigen Phase gelöst wird. Hieraus wird die Säure nach dem Ansäuern ausgefällt.

Liegt jedoch der Wirkstoff überwiegend als freies Cannabidiol vor, wird das Lösungsmittel abdestilliert und der Rückstand in einem polaren organischen Lösungsmittel aufgelöst, wobei unerwünschte lipophile Bestandteile ausfallen und abfiltriert werden. Der Wirkstoff wird dann durch Eindampfen des Filtrats gewonnen.

Ist in dem Lösungsmittelextrakt ein Gemisch von Cannabidiol-Säure und Cannabidiol enthalten, empfiehlt sich die Behandlung des Lösemittelextraktes zunächst mit einer wässrigen Alkalilösung und einer anschließenden Extraktion des in dem organischen Lösungsmittel zurückgebliebenen Cannabidiols mittels eines polaren organischen Lösungsmittels.

Die in den so gewonnenen Extrakten enthaltene Cannabidiolsäure wird anschließend durch Erhitzen decarboxyliert, dann das entstandene Cannabidiol im Vakuum schonend destilliert und zur Kristallisation gebracht. Die Decarboxylierung erfolgt unter Ausschluss von Sauerstoff, also entweder im Vakuum oder unter einem Schutzgas wie Stickstoff. Die Kristallisation des Cannabidiols wird durch Verreiben des Destillats z. B. mit Petroläther erleichtert.

Das so erhaltene Cannabidiol wird nach dem erfindungsgemäßen Verfahren dann vorteilhafterweise mit Hilfe von Lewis-Katalysatoren, ggf. unter Zusatz basischer Trocknungsmittel zu Dronabinol zyklisiert. Als geeignete Lewis-Säuren werden wasserfreie Salze von Silber, Zinn, Zink oder Magnesium, z. B. Zinkchlorid, Zinkbromid, Zinkjodid. Zinktrifluormethansulfonat. Zinnchlorid, Zinnbromid, Zinnjodid, Zinntrifluormethansulfonat, Magnesiumchlorid, Magnesiumbromid, Magnesiumjodid, Magnesiumtrifluormethansulfonat, Silberchlorid, Silberbromid, Silberjodid, Silbertrifluormethansulfonat in einem organischen Lösungsmittel eingesetzt.

Das so erhaltene Rohdronabinol wird anschließend chromatographisch gereinigt. Hierzu wird eine Lösung des Rohdronabinols in einem organischen Lösungsmittel z.B. über eine Kieselgelsäule gegeben. Nach Entfernung des Lösungsmittels aus dem Eluat wird der Rückstand anschließend einer Hochvakuumdestillation unterworfen, wobei sich eine Kurzwegdestillation oder eine Destillation im Kugelrohr, vorzugsweise unter Zusatz von Basen, besonders bewährt haben.

Das hierbei gewonnene Endprodukt ist hochreines Dronabinol, das bei Zimmertemperatur eine viskose Flüssigkeit ist, sich jedoch bei höherer Temperatur verflüssigt und z. B. bei Temperatur von 60 bis 80°C ohne weiteres in eine kalibrierte, gasdichte Glasspritze einfüllen lässt. Diese mit dem Dronabinol gefüllte Glasspritze wird an Apotheken ausgeliefert, die nach gelinder Erwärmung der Glasspritze den verflüssigten Inhalt genau dosieren und daraus verschiedenartige Arzneimittel herstellen können, z. B. flüssige Arzneimittel durch Zugabe genau abgemessener Mengen Dronabinols z. B. zu Sesamöl oder anderen Speiseölen, mit Dronabinol gefüllte Gelatinekapseln, in denen das Dronabinol von Oleum Cacao aufgenommen und in den Kapseln fixiert wird oder auch Inhalatlösungen, in denen das Dronabinol in Alkohol gelöst wird.

Das so gewonnene Dronabinol lässt sich auch aus einer alkoholischen Lösung mit Hilfe eines speziellen Inhalators (z. B. Inhalator Vulcano der Fa. Vapormed) so applizieren, dass der Wirkstoff mit Hilfe eines heißen Luftstromes in einem geschlossenen Beutel verdampft wird, aus dem es als Aerosol inhaliert werden kann. Durch diese Form der Applikation ist eine besonders hohe Bioverfügbarkeit gewährleistet. Durch Zusatz von weiteren Stoffen wie Cannabidiol und/oder ätherischen Ölen und/oder Lokalanästhetika kann die Bioverfügbarkeit weiter verbessert werden, da die Reizung der Luftwege bei der Inhalation gesenkt wird und so eine längere Verweilzeit des Aerosols in der Lunge möglich ist.

Das erfiridungsgemäße Verfahren ist durch eine Reihe von beachtlichen Vorteilen ausgezeichnet. Zunächst einmal ist zu betonen, dass der als Ausgangsmaterial eingesetzte Faserhanf ohne Probleme angebaut werden kann, da er keinen gesetzlichen Beschränkungen unterliegt.

Verfahrensmäßig ist ein besonderer Vorteil darin zu sehen, dass der Einsatz eines bereits Wirkstoffe enthaltenden organischen Lösungsmittels zur Extraktion des Pflanzenmaterials zu einer überaus erwünschten Erhöhung der Wirkstoffkonzentration führt, ohne dass bei der Extraktgewinnung eine Belastung durch thermische Konzentrierung erfolgt. Hierdurch wird die Decarboxylierung der Cannabidiol-Säure weitgehend verhindert. Die Extraktion der Cannabidiol-Säure aus dem Lösungsmittelextrakt mit Hilfe von wässrigen Alkali, die zweckmäßigerweise durch Gegenstromextraktion zu einer selektiven Anreicherung der Wirkstoffe führt, reduziert gleichzeitig auch den Gehalt an etwa vorhandenen Pestiziden erheblich.

Ein weiterer Vorteil ist darin zu sehen, dass die thermische Decarboxylierung des aus dem Pflanzenmaterial gewonnenen Extraktrückstandes Probleme verhindert, die bei der destillativen Aufarbeitung des Rohrproduktes durch eine dann erfolgende Gasentwicklung entstehen würden.

Ein weiterer verfahrensmäßiger Vorteil lässt sich dadurch erreichen, dass man das durch Zyklisierung von Cannabidiol gewonnene Dronabinol zunächst durch Destillation und Kristallisation reinigt und damit die anschließende chromatographische Aufarbeitung erleichtert.

Bei allen Synthesen des Δ9-Tetrahydrocannabinols muss darauf geachtet werden, dass das thermodynamisch stabilere, aber für die medizinische Anwendung uninteressante Δ8-Tetrahydrocannabinol nicht entsteht. Die Gefahr einer Bildung des Δ8-Tetrahydrocannabinols besteht grundsätzlich auch bei der erfindungsgemä-ßen Zyklisierung mit BF3-Etherat, jedoch kann diese unerwünschte Reaktion verhindert werden, wenn bei der Zyklisierung ein basisches Trocknungsmittel eingesetzt wird.

Noch vorteilhafter ist die Verwendung schwacher Lewis-Säuren, weil dann die Bildung des Δ8-Tetrahydrocannabinols deutlich verringert wird.

Eine besonders wichtige Ausgestaltung der vorliegenden Erfindung ist in dem Abfüllen des hochgereinigten Dronabinols in Spritzen zu sehen, aus denen problemlos kleinere Mengen Dronabinols für Rezepturzwecke entnommen werden können, ohne dass die Restmengen der sauerstoffempfindlichen Substanz mit Luft in Berührung kommen.

Das erfindungsgemäße Verfahren eröffnet somit einen neuen und einfachen Zugang zu dem für medizinische Zwecke immer wichtiger werdenden Dronabinol.

### Herstellung von Dronabinol

### Beispiel 1

Zu einer Lösung von 5 g Cannabidiol in 1.600 ml Methylenchlorid wird unter Rühren 5 ml Bortrifluoridetherat zugegeben und die Lösung bei Raumtemperatur stehen gelassen. Nach beendeter Reaktion wird mit der Zugabe von 500 ml Wasser gestoppt. Der nach dem Abdestillieren des Lösungsmittel entstehende Rückstand enthält

| | |
|---|---|
| Cannabidiol | 1,14 % |
| Iso-Tetrahydrocannabinol | 18,67 % |
| Δ8-Tetrahydrocannabinol | 5,59% |
| Dronabinol | 74,6 %. |

Das Gemisch wird mittels präparativer HPLC in seine Bestandteile aufgetrennt und das gereinigte Dronabinol einer Vakuumdestillation unterzogen.

### Beispiel 2

Zu einer Lösung von 5 g Cannabidiol in 1.600 ml Methylenchlorid und 5 g Kaliumcarbonat wird unter Rühren 5 ml Bortrifluoridetherat zugegeben und die Lösung bei Raumtemperatur stehen gelassen. Nach beendeter Reaktion wird mit der Zugabe von 500 ml Wasser gestoppt. Der nach dem Abdestillieren des Lösungsmittel entstehende Rückstand enthält

| | |
|---|---|
| Cannabidiol | 1,00% |
| Iso-Tetrahydrocannabinol | 13,87% |
| Δ8-Tetrahydrocannabinol | 1,59% |
| Dronabinol | 82.4 % |

Die Aufarbeitung erfolgt entsprechend Beispiel 1.

### Beispiel 3

Eine Lösung von 5 g Cannabidiol in 1.600 ml Dichlorethan mit 10 g Zinkchlorid wird 24 Stunden am Rückfluss gekocht. Nach beendeter Reaktion wird mit der Zugabe von 500 ml Wasser gestoppt. Der nach dem Abdestillieren des Lösungsmittels entstehende Rückstand enthält

| | |
|---|---|
| Cannabidiol | 2.87% |
| Iso-Tetrahydrocannabinol | 5,75% |
| Δ8-Tetrahydrocannabinol | 5,01% |
| Dronabinol | 86,37% |

Die Aufarbeitung erfolgt entsprechend Beispiel 1.

## Patentansprüche

1. Verfahren zur Synthese von Dronabinol, **dadurch gekennzeichnet, dass** man
- als Ausgangsstoffe für die Synthese von Dronabinol, Cannabidiolsäure und/oder Cannabidiol verwendet,
- das gegebenenfalls durch Decarboxylierung gewonnene Cannabidiol mit Hilfe von Lewis-Katalysatoren zu Dronabinol zyklisiert, um Rohdronabinol zu erhalten.

2. Verfahren nach Anspruch 1, **dadurch gekennzeichnet, dass** als Ausgangsmaterial Faserhanf vorgesehen ist.

3. Verfahren nach den Ansprüchen 1 oder 2, **dadurch gekennzeichnet, dass** man das Rohdronabinol durch ein chromatographisches Verfahren reinigt.

4. Verfahren nach den Ansprüchen 1 bis 3, **dadurch gekennzeichnet, dass** man den aus dem Eluat nach Abdestillation des Lösungsmittels gewonnene Rückstand durch Vakuumdestillation reinigt.

5. Verfahren zur Herstellung von Dronabinol, **dadurch gekennzeichnet, dass** man
a) Cannabidiol oder Cannabidiolsäure aus Faserhanf isoliert,
b) das ggf. durch Decarboxylierung gewonnene Cannabidiol in Gegenwart von Lewis-Sauren in einem nicht polaren Lösungsmittel zum Dronabinol zyklisiert,
c) dieses durch ein chromatographisches Verfahren isoliert und
d) den aus dem Eluat nach Abdestillation des Lösungsmittels gewonnenen Rückstand durch Vakuumdestillation reinigt.

6. Verfahren nach den Ansprüchen 1 bis 5, **dadurch gekennzeichnet, dass** man als Lewis - Katalysatoren wasserfreie Salze von Silber, Zinn, Zink oder Magnesium, z. B. Zinkchlorid, Zinkbromid, Zinkjodid. Zinktrifluormethansulfonat. Zinnchlorid, Zinnbromid, Zinnjodid, Zinntrifluormethansulfonat, Magnesiumchlorid, Magnesiumbromid, Magnesiumjodid, Magnesiumtrifluormethansulfonat, Silberchlorid, Silberbromid, Silberjodid, Silbertrifluormethansulfonat in einem organischen Lösungsmittel einsetzt.

7. Verfahren nach den Ansprüchen 1 bis 6, **dadurch gekennzeichnet, dass** man aus den Blüten oder Blättern des Faserhanfs die wirkstoffhaltigen Anteile extrahiert.

8. Verfahren nach den Ansprüchen 1 bis 7, **dadurch gekennzeichnet, dass** man aus den durch Sieben gewonnenen Bestandteilen die Wirkstoffe durch Extraktion mit einem nicht-polaren Lösungsmittel isoliert.

9. Verfahren nach den Ansprüchen 1 bis 8, **dadurch gekennzeichnet, dass** man die Wirkstoffe in dem Lösungsmittel durch Extraktion frischen Pflanzenmaterials mit einem bereits Wirkstoffe enthaltenden Lösungsmittel ohne Abdestillation des Lösungsmittels anreichert.

10. Verfahren nach den Ansprüchen 1 bis 9, **dadurch gekennzeichnet, dass** man zur Reinigung der Wirkstoffe
a) den Extrakt mit wässriger Lauge behandelt, aus der nach dem Ansäuern die freien Carbonsäuren ausgefällt werden und/oder
b) das Lösungsmittel abdestilliert und den Rückstand in einem polaren, organischen Lösungsmittel auflöst, wobei unerwünschte lipophile Bestandteile ausfallen und abfiltriert werden, und dann den im Filtrat enthaltenen Wirkstoff durch Eindampfen gewinnt,
den nach a) und/oder b) gewonnenen Rückstand durch Erhitzen decarboxyliert, dann im Vakuum destilliert und das entstandene Cannabidiol zur Kristallisation bringt.

11. Verfahren nach den Ansprüchen 1 bis 10, **dadurch gekennzeichnet, dass** man das Cannabidiol durch Zusatz von BF3-Etherat, ggf. in Gegenwart eines alkalischen Trocknungsmittels in einem organischen Lösungsmittel zu Dronabinol zyklisiert.

12. Verfahren nach den Anspruch 11, **dadurch gekennzeichnet, dass** man als alkalisches Trockenmittel Kaliumcarbonat verwendet.

13. Verfahren nach den Ansprüchen 1 bis 12, **dadurch gekennzeichnet, dass** man das Cannabidiol durch Zusatz schwacher Lewis-Säuren in einem organischen Lösungsmittel zu Dronabinol zyklisiert.

14. Verfahren nach den Ansprüchen 12 und 13, **dadurch gekennzeichnet, dass** man die Dronabinol enthaltende Lösung auf einer Kieselgelsäule chromatographiert.

15. Verfahren nach den Ansprüchen 10 bis 14, **dadurch gekennzeichnet, dass** man den aus dem Eluat gewonnenen Rückstand von Dronabinol durch eine Vakuumdestilation vorzugsweise eine Hochvakuumdestillation reinigt.

16. Inhalationslösung zur thermischen Vernebelung mit heißer Luft, **dadurch gekennzeichnet, dass** sie Dronabinol hergestellt gemäß einem der Ansprüche 1 bis 14 enthält.

17. Inhalationslösung nach Anspruch 16, **dadurch gekennzeichnet, dass** sie zusätzlich Cannabidiol, ein Lokalanästhetikum und/oder ätherische Öle enthält.

18. Verfahren zur Herstellung eines Dronabinol enthaltenden Arzneimittels, **dadurch gekennzeichnet, dass** in einem ersten Schritt in eine kalibrierte, gasdichte Glasspritze Dronabinol eingefüllt wird und in einem zweiten Schritt eine dosierte Abgabe des verflüssigten Inhaltes erfolgt, wobei die Abgabe des Inhalts erfolgt, ohne dass die eingefüllte Restmenge Dronabinols mit Luft in Berührung kommt.

19. Verfahren nach Anspruch 18, **dadurch gekennzeichnet, dass** das Dronabinol nach einem Verfahren der Ansprüche 1-15 gewonnen wird.

## Claims

1. Process for synthesizing dronabinol, **characterized in that**
- the starting materials used for the synthesis of dronabinol are cannabidiolic acid and/or cannabidiol,
- the cannabidiol which may have been obtained by decarboxylation is cyclized with the aid of Lewis catalysts to give dronabinol, in order to obtain crude dronabinol.

2. Process according to Claim 1, **characterized in that** the starting material envisaged is fibre hemp.

3. Process according to Claim 1 or 2, **characterized in that** the crude dronabinol is purified by a chromatographic process.

4. Process according to Claims 1 to 3, **characterized in that** the residue obtained from the eluate after distilling off the solvent is purified by vacuum distillation.

5. Process for preparing dronabinol, **characterized in that**
a) cannabidiol or cannabidiolic acid is isolated from fibre hemp,
b) the cannabidiol which may have been obtained by decarboxylation is cyclized in the presence of Lewis acids in a nonpolar solvent to give dronabinol,
c) the latter is isolated by a chromatographic process and
d) the residue obtained from the eluate after the solvent has been distilled off is purified by vacuum distillation.

6. Process according to Claims 1 to 5, **characterized in that** the Lewis catalysts used are anhydrous salts of silver, tin, zinc or magnesium, e.g. zinc chloride, zinc bromide, zinc iodide, zinc trifluoromethanesulphonate, tin chloride, tin bromide, tin iodide, tin trifluoromethanesulphonate, magnesium chloride, magnesium bromide, magnesium iodide, magnesium trifluoromethanesulphonate, silver chloride, silver bromide, silver iodide, silver trifluoromethanesulphonate, in an organic solvent.

7. Process according to Claims 1 to 6, **characterized in that** the active ingredient-containing fractions are extracted from the flowers or leaves of fibre hemp.

8. Process according to Claims 1 to 7, **characterized in that** the active ingredients are isolated from the constituents obtained by screening by extraction with a nonpolar solvent.

9. Process according to Claims 1 to 8, **characterized in that** the active ingredients are accumulated in the solvent by extracting fresh plant material with a solvent which already comprises active ingredients without distilling off the solvent.

10. Process according to Claims 1 to 9, **characterized in that** the active ingredients are purified by
a) treating the extract with aqueous alkali, from which the free carboxylic acids are precipitated after the acidification and/or
b) distilling off the solvent and dissolving the residue in a polar organic solvent to precipitate out undesired lipophilic constituents which are filtered off, and then obtaining the active ingredient present in the filtrate by evaporative concentration,
decarboxylating the residue obtained in a) and/or b) by heating, then distilling it under reduced pressure and crystallizing the cannabidiol formed.

11. Process according to Claims 1 to 10, **characterized in that** the cannabidiol is cyclized to dronabinol by adding BF3 etherate, optionally in the presence of an alkaline desiccant in an organic solvent.

12. Process according to Claim 11, **characterized in that** the alkaline desiccant used is potassium carbonate.

13. Process according to Claims 1 to 12, **characterized in that** the cannabidiol is cyclized to dronabinol by adding weak Lewis acids in an organic solvent.

14. Process according to Claims 12 and 13, **characterized in that** the solution comprising dronabinol is chromatographed on a silica gel column.

15. Process according to Claims 10 to 14, **characterized in that** the residue of dronabinol obtained from the eluate is purified by a vacuum distillation, preferably a high-vacuum distillation.

16. Inhalation solution for thermal nebulization with hot air, **characterized in that** it comprises dronabinol prepared according to one of Claims 1 to 14.

17. Inhalation solution according to Claim 16, **characterized in that** it additionally comprises cannabidiol, a local anaesthetic and/or essential oils.

18. Process for producing a medicament comprising dronabinol, **characterized in that**, in a first step, dronabinol is introduced into a calibrated gas-tight glass syringe and, in a second step, the liquefied contents are delivered in a controlled dosage, the ingredients being delivered without the remaining amount of dronabinol introduced coming into contact with air.

19. Process according to Claim 18, **characterized in that** the dronabinol is obtained by a process of Claims 1-15.

## Revendications

1. Procédé pour la synthèse de dronabinol, **caractérisé**
- **en ce qu'**on utilise comme substance de départ pour la synthèse du dronabinol, de l'acide cannabidiolique et/ou du cannabidiol,
- **en ce qu'**on cyclise le cannabidiol le cas échéant obtenu par décarboxylation à l'aide de catalyseurs de Lewis en dronabinol, pour obtenir du dronabinol brut.

2. Procédé selon la revendication 1, **caractérisé en ce qu'**on prévoit, comme matière de départ, le chanvre à fibres.

3. Procédé selon les revendications 1 ou 2, **caractérisé**
**en ce qu'**on purifie le dronabinol brut par un procédé chromatographique.

4. Procédé selon les revendications 1 à 3, **caractérisé**
**en ce qu'**on purifie le résidu obtenu à partir de l'éluat après élimination par distillation du solvant par une distillation sous vide.

5. Procédé pour la préparation de dronabinol, **caractérisé**
a) **en ce qu'**on isole le cannabidiol ou l'acide cannabidiolique à partir de chanvre à fibre,
b) **en ce qu'**on cyclise le cannabidiol le cas échéant obtenu par décarboxylation en présence d'acides de Lewis dans un solvant non polaire en dronabinol,
c) **en ce qu'**on isole celui-ci par un procédé chromatographique et
d) **en ce qu'**on purifie le résidu obtenu à partir de l'éluat après élimination par distillation du solvant par une distillation sous vide.

6. Procédé selon les revendications 1 à 5, **caractérisé**
**en ce qu'**on utilise comme catalyseurs de Lewis des sels anhydres de l'argent, de l'étain, du zinc ou du magnésium, par exemple le chlorure de zinc, le bromure de zinc, l'iodure de zinc, le trifluorométhanesulfonate de zinc, le chlorure d'étain, le bromure d'étain, l'iodure d'étain, le trifluorométhanesulfonate d'étain, le chlorure de magnésium, le bromure de magnésium, l'iodure de magnésium, le trifluorométhanesulfonate de magnésium, le chlorure d'argent, le bromure d'argent, l'iodure d'argent, le trifluorométhanesulfonate d'argent dans un solvant organique.

7. Procédé selon les revendications 1 à 6, **caractérisé**
**en ce qu'**on extrait les proportions contenant les substances actives à partir des fleurs ou des feuilles du chanvre à fibre.

8. Procédé selon les revendications 1 à 7, **caractérisé**
**en ce qu'**on isole les substances actives à partir des constituants obtenus par tamisage par extraction avec un solvant non polaire.

9. Procédé selon les revendications 1 à 8, **caractérisé**
**en ce qu'**on enrichit les substances actives dans le solvant par extraction de matière végétale fraîche avec un solvant contenant déjà des substances actives sans élimination par distillation du solvant.

10. Procédé selon les revendications 1 à 9, **caractérisé**
**en ce que**, pour la purification des substances actives
a) on traite l'extrait avec de la lessive aqueuse, à partir de laquelle les acides carboxyliques libres sont précipités après acidification et/ou
b) on élimine le solvant par distillation et on dissout le résidu dans un solvant polaire, organique, les constituants lipophiles non souhaités précipitant et étant séparés par filtration, et on obtient ensuite la substance active contenue dans le filtrat par concentration par évaporation,
on décarboxyle le résidu obtenu selon a) et/ou b) par chauffage, puis on distille sous vide et on amène le cannabidiol formé à cristalliser.

11. Procédé selon les revendications 1 à 10, **caractérisé**
**en ce qu'**on cyclise le cannabidiol par addition d'éthérate de BF3, le cas échéant en présence d'un dessiccateur alcalin dans un solvant organique en dronabinol.

12. Procédé selon la revendication 11, **caractérisé en ce qu'**on utilise comme dessiccateur alcalin du carbonate de potassium.

13. Procédé selon les revendications 1 à 12, **caractérisé**
**en ce qu'**on cyclise le cannabidiol par addition d'acides de Lewis faibles dans un solvant organique en dronabinol.

14. Procédé selon les revendications 12 et 13, **caractérisé**
**en ce qu'**on soumet la solution contenant le dronabinol sur une colonne de gel silicique.

15. Procédé selon les revendications 10 à 14, **caractérisé**
**en ce qu'**on purifie le résidu de dronabinol obtenu à partir de l'éluat par une distillation sous vide, de préférence une distillation sous un vide poussé.

16. Solution d'inhalation pour l'atomisation thermique avec de l'air chaud, **caractérisée**
**en ce qu'**elle contient du dronabinol préparé selon l'une quelconque des revendications 1 à 14.

17. Solution d'inhalation selon la revendication 16, **caractérisée**
**en ce qu'**elle contient en outre du cannabidiol, un anesthésique local et/ou des huiles essentielles.

18. Procédé pour la préparation d'un médicament contenant du dronabinol, **caractérisé**
**en ce qu'**on introduit dans une première étape, dans une seringue en verre calibrée, imperméable du gaz, du dronabinol et on réalise, dans une deuxième étape, une émission dosée du contenu liquéfié, l'émission du contenu étant réalisée sans que la quantité résiduelle introduite de dronabinol n'entre en contact avec l'air.

19. Procédé selon la revendication 18, **caractérisé en ce que** le dronabinol est obtenu selon un procédé des revendications 1-15.
